# EUROPEAN PATENT SPECIFICATION

(11) **EP 0 902 790 B1**
(45) Date of publication and mention of the grant of the patent: **16.08.2000**
(21) Application number: 97918091.6
(22) Date of filing: 26.03.1997
(51) Int. Cl.: C07J 73/00, C07J 41/00

(54) **PROCESS FOR PREPARING STEROIDS HAVING A CARBOXAMIDE SIDE-CHAIN**
VERFAHREN ZUR HERSTELLUNG VON STEROID DERIVATE MIT EINER CARBOXAMID DERIVIERTEN SEITENKETTE
PROCEDE DE PREPARATION DE STEROIDES A CHAINE LATERALE CARBOXAMIDE

(30) Priority: 18.04.1996 GB 9608045
(43) Date of publication of application: 24.03.1999
(73) Proprietor: PHARMACIA & UPJOHN S.p.A., 20152 Milano (IT); Panzeri, Achille, 22055 Merate (IT); Nesi, Marcella, 20146 Milan (IT)
(72) Inventor: PANZERI, Achille, I-22055 Merate (IT); NESI, Marcella, I-20146 Milan (IT)
(86) International application number: EP9701626
(87) International publication number: WO9740062

(56) References cited:
- WO-A-93/14107
- US-A- 4 348 327

## Description

The present invention relates to a process for preparing steroids having a carboxamide side-chain. More particularly, the present invention relates to a process for preparing steroids of the general formula: 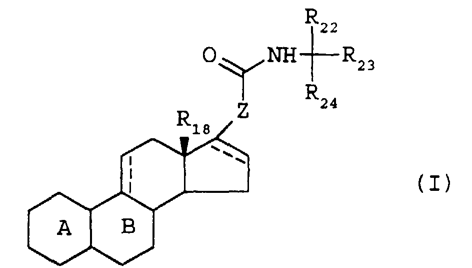 wherein:
the symbols --- are, each independently, single or double bonds;
Z is a single bond, or a straight or branched C₁-C₅ alkylene; the moiety 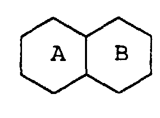 represents the A and B rings of a steroid;
R₁₈ is hydrogen or C₁-C₄ alkyl;
one of R₂₂, R₂₃, and R₂₄ is an optionally substituted C₆₋₁₀ aryl group and the other two are C₁₋₃ perfluoroalkyl groups.

Particularly, the moiety 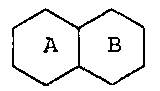 may be selected from:
1) 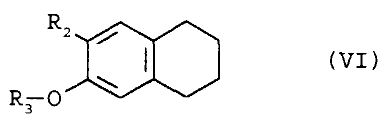 wherein: R₃ is hydrogen or C₁-C₄ alkyl; and R₂ is hydrogen or -OR₂', wherein R₂' is hydrogen or C₁-C₄ alkyl;
2) 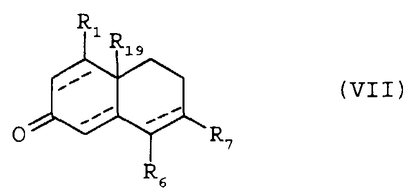 wherein: the symbols --- are, each independently, single or double bonds; R₁, R₆, R₇, and R₁₉ are, each independently, hydrogen or C₁-C₄ alkyl;
3) 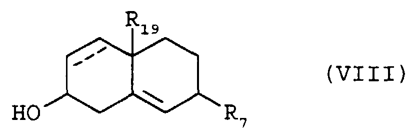 wherein: the symbol --- is a single or a double bond; R₇ is hydrogen or C₁-C₄ alkyl; and R₁₉ is hydrogen or C₁-C₄ alkyl;
4) 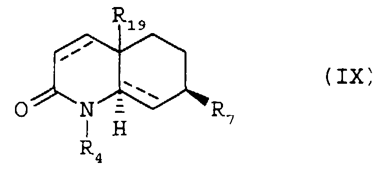 wherein: the symbols --- are, each independently, single or double bonds; R₄ is hydrogen, C₁-C₄ alkyl, C₆-C₁₀ aryl, C₇-C₁₀ arylalkyl, acetyl, benzoyl, or tosyl; R₇ is hydrogen or C₁-C₄ alkyl; R₁₉ is hydrogen or C₁-C₄ alkyl;
5) 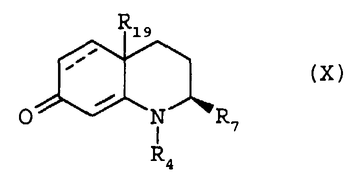 wherein: the symbol --- is a single or a double bond; R₄ is hydrogen, C₁-C₄ alkyl, C₆-C₁₀ aryl, C₇-C₁₀ arylalkyl, acetyl, benzoyl, or tosyl; R₇ is hydrogen or C₁-C₄ alkyl; R₁₉ is hydrogen or C₁-C₄ alkyl;
6) 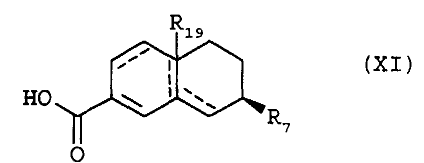 wherein: the symbols --- are, each independently, single or double bonds; R₁₉ is hydrogen, C₁-C₄ alkyl, or it is absent when linked to a double-bonded carbon atom; R₇ is hydrogen or C₁-C₄ alkyl;
7) 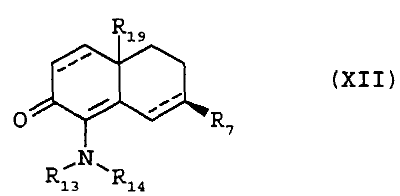 wherein: the symbols --- are, each independently, single or double bonds; R₇ is hydrogen or C₁-C₄ alkyl; C₁₉ is hydrogen or C₁-C₄ alkyl; R₁₃ and R₁₄ are, each independently, hydrogen, C₁-C₄ alkyl, C₆-C₁₀ aryl, C₇-C₁₀ arylalkyl, acetyl, benzoyl, tosyl or, taken together, phthalyl.

The steroid compounds of formula (I) are known as pharmacologically active products. For example, the compounds of formula (I) wherein the AB ring moiety has formula (VII) are reported to be testosterone 5α-reductase inhibitors (see, e.g., U.S. Patents No. 4,191,759, 4,220,775, and 4,377,584). The compounds of formula (I) wherein the AB ring moiety has formula (IX) are reported to be testosterone 5α-reductase inhibitors (see, e.g., EP-4949, EP-155046, WO 94/20104, EP-484094, EP-200859, WO 94/03475, WO 95/07927, EP-277002; J. Med. Chem. 27, 1690-1701 (1984) and 29, 2298-2315 (1986)). The compounds of formula (I) wherein the AB ring moiety has formula (X) are reported to be testosterone 5α-reductase inhibitors (see, for example, WO 93/13124; J. Med. Chem. 37, 2352-2360 (1994)). The compounds of formula (I) wherein the AB ring moiety has formula (XI) are reported to be testosterone 5α-reductase inhibitors (see, for example, EP-289327, EP-567271; J. Med. Chem. 33, 937-942 and 943-950 (1990)). The compounds of formula (I) wherein the AB ring moiety has formula (XII) are reported to be testosterone 5α-reductase inhibitors (see, for example, EP-469548, EP-469549).

The compounds of formula (I) are usually prepared by condensation reaction of the corresponding 17-carboxylic acid or derivative thereof, such as for example a chloride, a pyridyl thioester, an imidazole or a hydroxybenzotriazole derivative, with a suitable amine.
Such process shows some drawbacks, especially when the amine that has to be condensed with the carboxylic acid is scarcely reactive, because of its sterical hindrance or its poor nucleophilicity, or it is not readily available by synthesis. For example, in the case of the reaction between 3-oxo-4-aza-5α-androst-1-ene-17β-carboxylic acid and 1,1,1,3,3,3-hexafluoro-2-phenyl-2-propyl amine the corresponding amide is obtained with a yield of about 20% at most. On the contrary, the reaction between 17-cyano-4-aza-5α-androst-1-en-3-one and 1,1,1,3,3,3,-hexafluoro-2-phenyl-2-propyl triflate according to the present invention provides the amide with a yield of about 40%.

The Applicant has now found that the steroids of formula (I) having a carboxamide side-chain can be advantageously prepared by reacting the corresponding 17-cyanosteroids with a suitable alcohol or one of its activated derivative as defined hereinunder.

Therefore, the present invention provides a process for preparing a compound of formula: 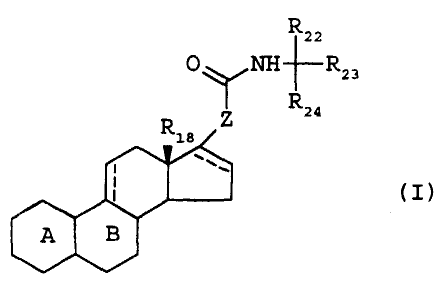 wherein:
the symbols --- are, each independently, single or double bonds;
Z is a single bond, or a straight or branched C₁-C₅ alkylene;
the moiety 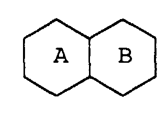 represents the A and B rings of a steroid;
R₁₈ is hydrogen or C₁-C₄ alkyl;
R₂₂, R₂₃, and R₂₄ are as previously defined; said process comprising reacting a compound of formula: 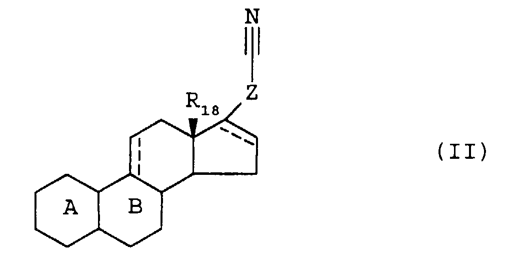 wherein the symbols --- , Z, R₁₈, and the moiety 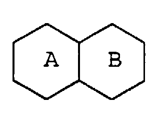 are defined as above;
with a compound of formula: 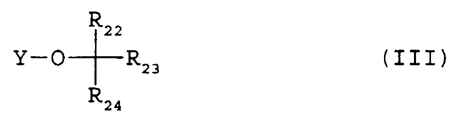 wherein R₂₂, R₂₃, and R₂₄ are defined as above, and Y is a trifluoromethanesulfonyl group.

In formula (I) and (II) R₁₈ is preferably hydrogen or methyl, while the moiety 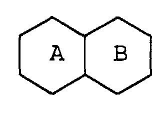 may be selected, e.g., from:
1) 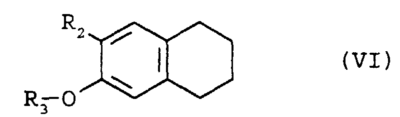 wherein: R₃ is hydrogen or C₁-C₄ alkyl; and R₂ is hydrogen or -OR₂', wherein R₂' is hydrogen or C₁-C₄ alkyl;
2) 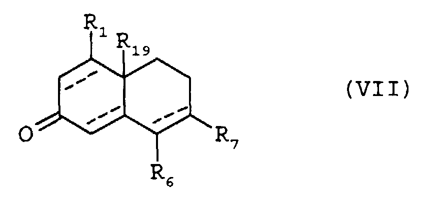 wherein: the symbols --- are, each independently, single or double bonds; R₁, R₆, R₇, and R₁₉ are, each independently, hydrogen or C₁-C₄ alkyl;
3) 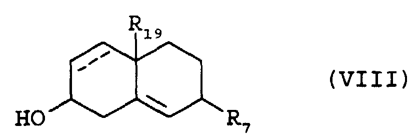 wherein: the symbol --- is a single or a double bond; R₇ is hydrogen or C₁-C₄ alkyl; and R₁₉ is hydrogen or C₁-C₄ alkyl;
4) 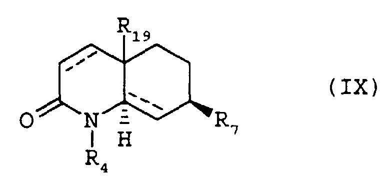 wherein: the symbols --- are, each independently, single or double bonds; R₄ is hydrogen, C₁-C₄ alkyl, C₆-C₁₀ aryl, C₇-C₁₀ arylalkyl, acetyl, benzoyl, or tosyl; R₇ is hydrogen or C₁-C₄ alkyl; R₁₉ is hydrogen or C₁-C₄ alkyl;
5) 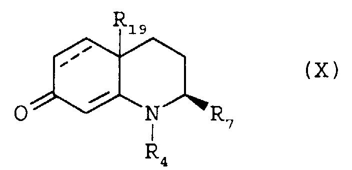 wherein: the symbol --- is a single or a double bond; R₄ is hydrogen, C₁-C₄ alkyl, C₆-C₁₀ aryl, C₇-C₁₀ arylalkyl, acetyl, benzoyl, or tosyl; R₇ is hydrogen or C₁-C₄ alkyl; R₁₉ is hydrogen or C₁-C₄ alkyl;
6) 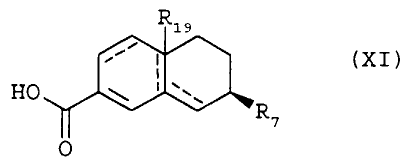 wherein: the symbols --- are, each independently, single or double bonds; R₁₉ is hydrogen, C₁-C₄ alkyl, or it is absent when linked to a double-bonded carbon atom; R₇ is hydrogen or C₁-C₄ alkyl;
7) 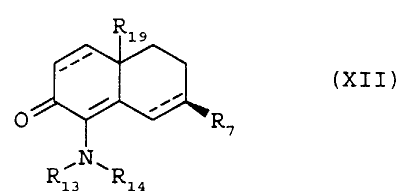 wherein: the symbols --- are, each independently, single or double bonds; R₇ is hydrogen or C₁-C₄ alkyl; C₁₉ is hydrogen or C₁-C₄ alkyl; R₁₃ and R₁₄ are, each independently, hydrogen, C₁-C₄ alkyl, C₆-C₁₀ aryl, C₇-C₁₀ arylalkyl, acetyl, benzoyl, tosyl or, taken together, phthalyl.

A C₁-C₅ alkylene may be e.g.: -CH₂-, -CH₂CH₂-, -CH₂CH₂CH₂-, -CH₂CH₂CH₂CH₂CH₂-, -CH(CH₃)-, -CH(CH₃)CH₂-, -CH(CH₃)CH₂CH₂-, or -CH(CH₃)CH₂CH₂CH₂-.
A C₁-C₄ alkyl may have a straight or branched chain; for example it may be: methyl, ethyl, n-propyl, iso-propyl, n-butyl, sec-butyl, iso-butyl, or tert-butyl.

Preferred C₁₋₃ perfluoroalkyl groups are e.g.: -CF₃, -CF₂CF₃, -CF₂CF₂CF₃, or -CF(CF₃)₂.

An optionally substituted C₆-C₁₀ aryl is, e.g.: phenyl or naphthyl, optionally mono- or di-substituted by: halogen (preferably chlorine or fluorine), C₁-C₄ alkyl (preferably methyl, ethyl, n-propyl, n-butyl, iso-butyl), trifluoromethyl, cyano, methoxy, ethoxy, and/or nitro. Preferred optionally substituted C₆-C₁₀ aryls are, for example: phenyl, naphthyl, p-chlorophenyl, p-fluorophenyl, p-trifluorophenyl, p-cyanophenyl, p-methylphenyl, p-ethylphenyl, p-n-propylphenyl, p-n-butylphenyl, p-isobutylphenyl, p-methoxyphenyl, p-ethoxyphenyl, p-nitrophenyl, m-chlorophenyl, m-fluorophenyl, m-trifluorophenyl, m-cyanophenyl, m-methylphenyl, methylphenyl, m-n-propylphenyl, m-n-butylphenyl, m-isobutylphenyl, m-methoxyphenyl, m-ethoxyphenyl, m-nitrophenyl, o-chlorophenyl, o-fluorophenyl, o-trifluorophenyl, o-cyanophenyl, o-methylphenyl, o-ethylphenyl, o-n-propylphenyl, o-n-butylphenyl, o-isobutylphenyl, o-methoxyphenyl, 0-ethoxyphenyl, o-nitrophenyl, o,p-dimethylphenyl, o,p-difluorophenyl, o,p-dichlorophenyl, o,p-bistrifluoromethylphenyl, o,m-dimethylphenyl, o,m-difluorophenyl, o,m-dichlorophenyl, o,m-bistrifluoromethylphenyl, m,m-dimethylphenyl, m,m-dichlorophenyl, m,m-difluorophenyl, or m,m-bistrifluoromethylphenyl. Particularly preferred groups are: p-chlorophenyl, p-fluorophenyl, p-trifluorophenyl, p-cyanophenyl, p-methylphenyl, p-ethylphenyl, p-n-propylphenyl, p-n-butylphenyl, p-isobutylphenyl, p-methoxyphenyl, p-ethoxyphenyl, or p-nitrophenyl.

When the moiety 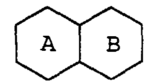 has formula (VI), the R₃ group is preferably: hydrogen, methyl or ethyl, and the group R₂ is preferably: hydrogen, hydroxy, methoxy, or ethoxy. Particularly preferred moieties of formula (VI) are the following:

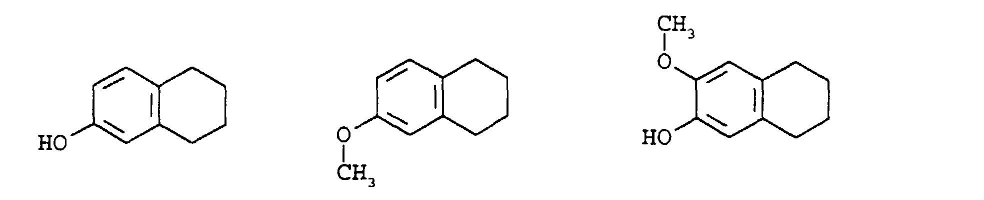

When the moiety 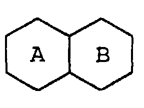 has formula (VII), the symbols --- may be single or double bonds, and the groups R₁, R₆, R₇ and R₁₉ are preferably, each independently, hydrogen or methyl. Particularly preferred moieties of formula (VII) are the following:

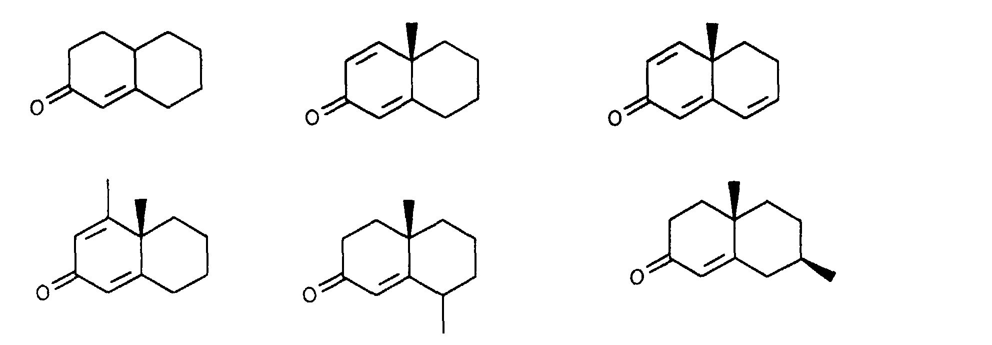

When the moiety 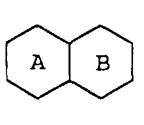 has formula (VIII), the symbol --- may be a single or a double bond, and the groups R₇ and R₁₉ are preferably, each independently, hydrogen or methyl.

Particularly preferred moieties of formula (VIII) are the following: 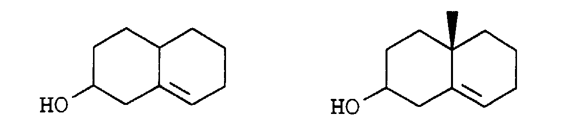 When the moiety 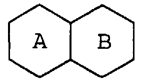 has formula (IX), the symbols --- may be, each independently, single or double bonds, the groups R₇ and R₁₉ are preferably, each independently, hydrogen or methyl, and the group R₄ is preferably: hydrogen, methyl, phenyl, benzyl, p-methoxyphenyl, acetyl, benzoyl, or tosyl. Particularly preferred moieties of formula (IX) are the following:

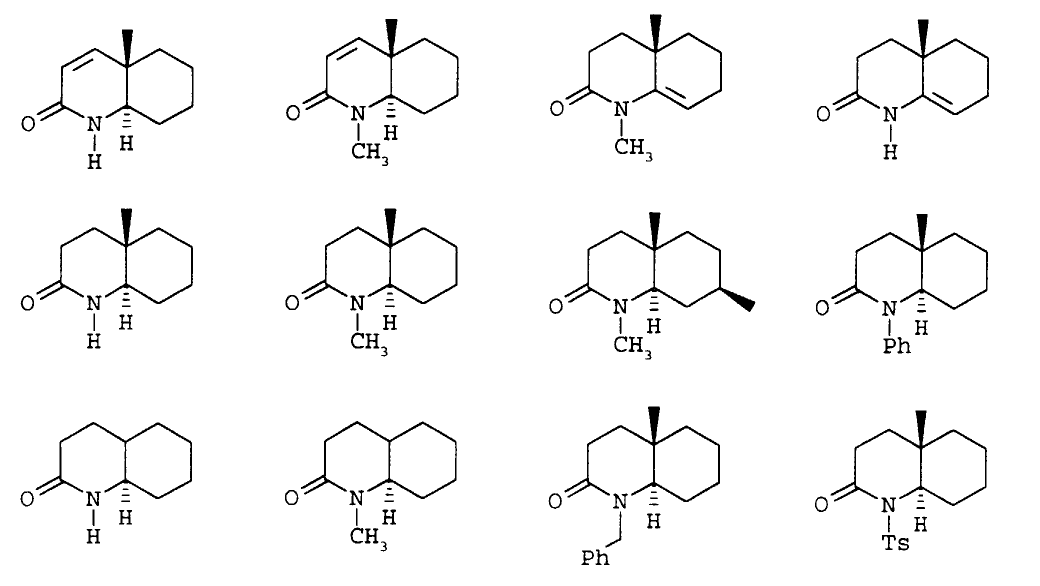

When the moiety 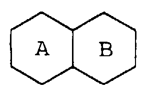 has formula (X), the symbol --- may be a single or a double bond, R₇ and R₁₉ are preferably, each independently, hydrogen or methyl, and R₄ is preferably: hydrogen, methyl, phenyl, benzyl, p-methoxyphenyl, acetyl, benzoyl, or tosyl. Particularly preferred moieties of formula (IX) are the following:

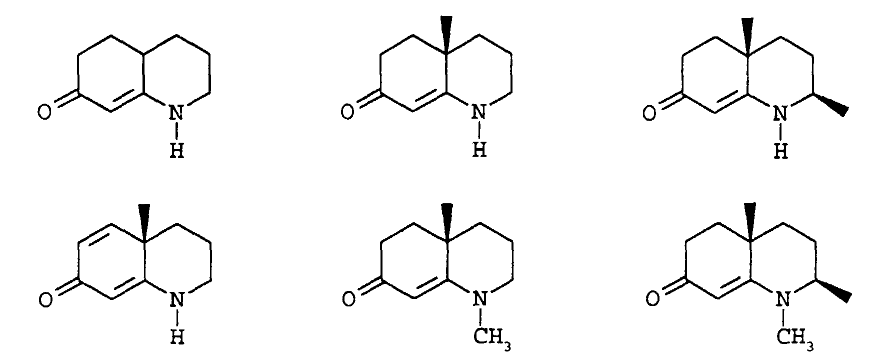

When the moiety 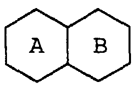 has formula (XI), the symbols --- may be, each independently, single or double bonds, R₇ is preferably hydrogen or methyl, and R₁₉ is preferably hydrogen or methyl, or it is absent when linked to a double-bonded carbon atom. Particularly preferred moieties of formula (XI) are the following:

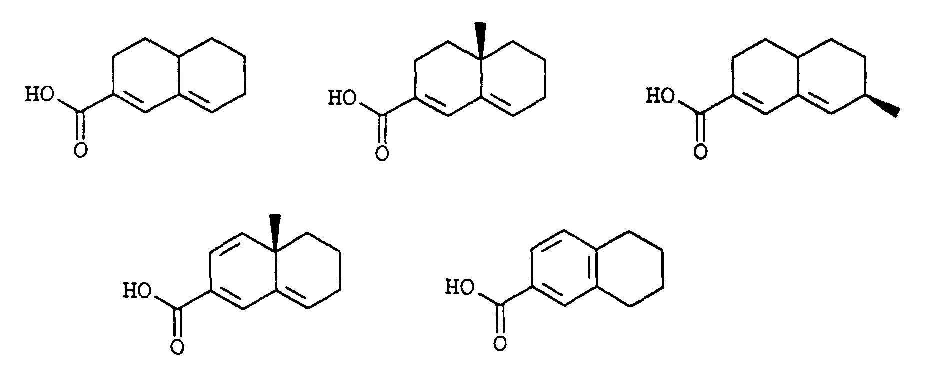

When the moiety 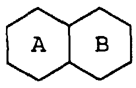 has formula (XII), the symbols --- may be, each independently, single or double bonds, R₇ and R₁₉ are preferably, each independently, hydrogen or methyl; and R₁₃ and R₁₄ are preferably, each independently, hydrogen, methyl, phenyl, benzyl, acetyl, benzoyl, or tosyl, or, taken together, phthalyl. Particularly preferred moieties of formula (XII) are the following:

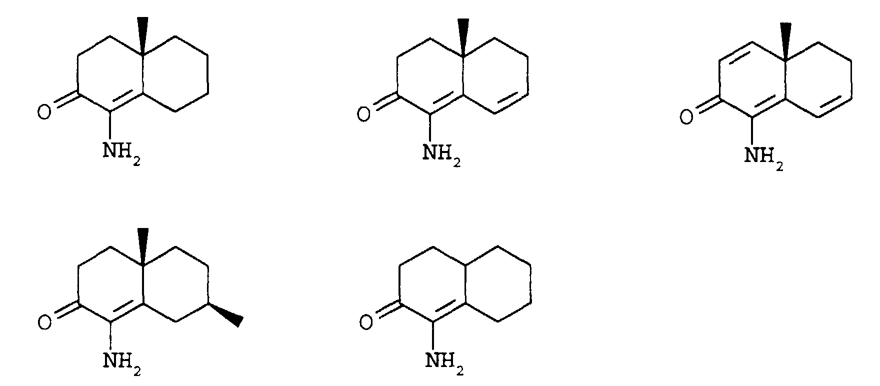

The process of the present invention can be employed to prepare both 17α and 17β epimers, however 17β epimers are preferred.
The process object of the present invention is advantageously carried out to prepare steroids of formula (I) wherein one of R₂₂, R₂₃ and R₂₄ is an optionally substituted C₆-C₁₀ aryl group, and the other two are C₁-C₃ perfluoroalkyl groups.

The process according to the present invention is preferably carried out to prepare one of the following steroids having a carboxamide side-chain:
1) N-(1,1,1,3,3,3-hexafluoro-2-phenylprop-2-yl)-3-oxo-4-aza-5α-androst-1-ene-17β-carboxamide;
2) N-(1,1,1,3,3,3-hexafluoro-2-phenylprop-2-yl)-3-oxo-4-aza-5α-androstane-17β-carboxamide;
3) N-(1,1,1,3,3,3-hexafluoro-2-phenylprop-2-yl)-4-methyl-3-oxo-4-aza-5α-androst-1-ene-17β-carboxamide;
4) N-(1,1,1,3,3,3-hexafluoro-2-phenylprop-2-yl)-4-methyl-3-oxo-4-aza-5α-androstane-17β-carboxamide;
5) N-(1,1,1,3,3,3-hexafluoro-2-phenylprop-2-yl)-3-oxo-4-aza-5α-androst-16-ene-17β-carboxamide;
6) N-(1,1,1,3,3,3-hexafluoro-2-phenylprop-2-yl)-4-methyl-3-oxo-4-aza-5α-androst-16-ene-17β-carboxamide;
7) N-(1,1,1,3,3,3-hexafluoro-2-phenylprop-2-yl)-3-oxo-androst-4-ene-17β-carboxamide;
8) N-(1,1,1,3,3,3-hexafluoro-2-phenylprop-2-yl)-3β-hydroxy-androst-5-ene-17β-carboxamide;
9) N-[1,1,1,3,3,3-hexafluoro-2-(p-methylphenyl)prop-2-yl]-3-oxo-4-aza-5α-androst-1-ene-17β-carboxamide;
10) N-[1,1,1,3,3,3-hexafluoro-2-(p-methylphenyl)prop-2-yl]-3-oxo-androst-4-ene-17β-carboxamide;
11) N-[1,1,1,3,3,3-hexafluoro-2-(p-fluorophenyl)prop-2-yl]-3-oxo-4-aza-5α-androst-1-ene-17β-carboxamide;
12) N-[1,1,1,3,3,3-hexafluoro-2-(p-fluorophenyl)prop-2-yl]-3-oxo-androst-4-ene-17β-carboxamide;
13) N-[1,1,1,3,3,3-hexafluoro-2-(p-chlorophenyl)prop-2-yl]-3-oxo-4-aza-5α-androst-1-ene-17β-carboxamide;
14) N-[1,1,1,3,3,3-hexafluoro-2-(p-chlorophenyl)prop-2-yl]-3-oxo-androst-4-ene-17β-carboxamide;
15) N-[1,1,1,3,3,3-hexafluoro-2-(p-trifluoromethyl)phenyl) prop-2-yl] 3-oxo-4-aza-5α-androst-1-ene-17β-carboxamide;
16) N-[1,1,1,3,3,3-hexafluoro-2-(p-trifluoromethylphenyl) prop-2-yl] 3-oxo-androst-4-ene-17β-carboxamide;
17) 17β-N-(1,1,1,3,3,3-hexafluoro-2-phenylprop-2-yl)carbamoyl-androsta-4,6-diene-3-carboxylate;
18) 17β-N-(1,1,1,3,3,3-hexafluoro-2-phenylprop-2-yl)carbamoyl-1,3,5(10)-estratriene-3-carboxylate;
19) N-(1,1,1,3,3,3-hexafluoro-2-phenylprop-2-yl)]-3-oxo-6-aza-androst-4-ene-17β-carboxamide; and
20) N-(1,1,1,3,3,3-hexafluoro-2-phenylprop-2-yl)-3-hydroxyestra-1,3,5(10)-triene-17β-carboxamide.

In general, the reaction of a nitrile with an alcohol, an alkene, or an alkyl or aryl halide to yield the corresponding amide is known in organic chemistry as Ritter reaction or its modifications (see e.g. J. J. Ritter and P. P. Minieri, J. Am. Chem. Soc. 70, 4045 (1948); L. I. Krimen and D. J. Cota, Organic Reaction 17, 213-325 (1969); A. L. J. Beckwith in J. Zabicky, "The Chemistry of amides", Wiley, New York, 1970, pp. 125-130; J. Casanova in Z. Rappoport, "The chemistry of the cyano group", Wiley, New York, 1970, pp. 913-915; D. Dopp and H. Dopp in "Methoden der Organischen Chemie (Huben-Weil)", vol. E5, pp. 1032-1041; R. Bishop in B. Trost, "Comprehensive Organic Synthesis", Pergamon Press, 1991, vol. 6, pp. 261-300; Synthesis 274-276 (1979); Tetr. Lett. 30 (5), 581-582 (1989)).

The process according to the present invention may be generally carried out by treating a mixture of a nitrile of formula (II) and a compound of formula (III), optionally in the presence of a solvent such as, for example, glacial acetic acid, acetic anhydride, di-n-butylether, chloroform, carbon tetrachloride, n-hexane, nitrobenzene, with a strong inorganic acid such as, for example, perchloric acid, phosphoric acid, 98% sulfuric acid, fluorosulfonic acid, or with a strong organic acid, such as, for example, trifluoromethanesulfonic acid, trifluoroacetic acid, at a temperature ranging from about room temperature to about the reflux temperature of the reaction mixture, for a time varying from about 30 minutes to about 8 hours, preferably in inert atmosphere of, for example, nitrogen or argon.

The process is generally carried out by adding to the mixture of the nitrile of formula (II) and the triflate of formula (III), as pure liquids or dissolved in a solvent, an organic acid such as, for example, trifluoroacetic acid or trifluoroethanol or trifluoromethanesulfonic acid or glacial acetic acid, and then stirring the mixture at a temperature ranging from about room temperature to the reflux temperature of the reaction mixture, preferably from 50° to 70°C, for a time varying from about 30 minutes to about 8 hours, in inert atmosphere of, for example, nitrogen. The reaction mixture is worked up by treatment with an aqueous alkaline solution (for example, a saturated sodium bicarbonate solution) and extracted with an organic solvent.

The starting compounds of formula (II) and (III) are known compounds and/or can be obtained by methods well known to anyone skilled in the art. Particularly, the compounds of formula (II) wherein the AB ring moiety has formula (VI) are disclosed e.g. in EP-A-675134; the compounds of formula (II) wherein the AB ring moiety has formula (VII) may be obtained from the corresponding 17-carboxylic acids described e.g. in U.S. Patents No. 4,191,759, 4,220,775 and 4,377,584; the compounds of formula (II) wherein the AB ring moiety has formula (VIII) are described e.g. in: Collection Czechoslov. 18, 407, 410, 412 (1953); Berichte 71, 1487-1492 (1938); the compounds of formula (II) wherein the AB ring moiety has formula (IX) are described e.g. in: EP-A-4949, EP-A-277002, J. Med. Chem. 27, 1690-1701 (1984) and 29, 2298-2351 (1986); the compounds of formula (II), wherein the AB ring moiety has formula (X) may be obtained from the corresponding 17-carboxylic acids described e.g in WO 93/13124 and J.Med.Chem. 37, 2352-2360 (1994); the compounds of formula (II) wherein the AB ring moiety has formula (XI) are described e.g. in EP-A-289327; the compounds of formula (II) wherein the AB ring moiety has formula (XII) may be obtained from the corresponding 17-carboxylic acids described e.g in EP-469,548 and EP-469,548.

The 17-cyanosteroids of formula (II) can be advantageously obtained by dehydration of the corresponding 17-carboxamides, according to the method reported in Synthesis 591-592 (1982). This synthetic route is especially advantageous for those compounds, such as the azasteroids, that cannot be subjected to severe dehydration conditions, such as chlorinating dehydratating agents in refluxing high-boiling solvents (e.g. thionyl chloride in dimethylformamide).

The following working examples are given to better illustrate the present invention, and cannot be construed as a limitation to the scope of the invention itself.

### EXAMPLE 1

### N-(1,1,1,3,3,3-hexafluoro-2-phenyl-2-propyl)-3-oxoandrost-4-ene-17β-carboxamide

[compound (I), wherein the moiety AB has formula (VII), wherein R₁=H, R₆=H, R₇=H and R₁₉=Me, the C₄-C₅ bond is a double bond, R₁₈=Me, Z=single bond, R₂₂=R₂₄=CF₃, R₂₃=Ph].

To a stirred mixture of 17β-cyanoandrost-4-en-3-one (100 mg, 0.335 mmol) and 1,1,1,3,3,3-hexafluoro-2-phenyl-2-propyl trifluoromethanesulfonate (252 mg, 0.669 mmol), under nitrogen atmosphere, trifluoroacetic acid (0.13 ml, 1.806 mmol) was added at room temperature. The mixture was then stirred at 60°C for 3 hrs. The reaction mixture was cooled in an ice bath, a saturated aqueous solution of sodium hydrogen carbonate (5 ml) was added, and the mixture was extracted with diethylether (3 x 10 ml). The combined organic extracts were washed with water until neutral, dried on sodium sulfate and the solvent was removed under vacuum. The crude product was purified by flash chromatography (eluant: n-hexane/ethyl acetate 70:30) to yield 72 mg (40%) of the title compound.
- NMR (CDCl₃) δ:: 0.77 (s, 3H, Me (18)), 1.29 (s, 3H, Me (19)), 5.72 (m, 1H, CH (4)), 5.93 (s, 1H, NH), 7.36-7.55 (m, 5H, Ph).

Following an analogous procedure, starting from the corresponding 17β-cyanosteroids and the suitable triflate, the compounds listed below were prepared:
N-(1,1,1,3,3,3-hexafluoro-2-phenylprop-2-yl)-3β-hydroxy-androst-5-ene-17β-carboxamide;
N-[1,1,1,3,3,3-hexafluoro-2-(p-methylphenyl)prop-2-yl]-3-oxo-androst-4-ene-17β-carboxamide;
N-[1,1,1,3,3,3-hexafluoro-2-(p-fluorophenyl)prop-2-yl]-3-oxo-androst-4-ene-17β-carboxamide;
N-[1,1,1,3,3,3-hexafluoro-2-(p-chlorophenyl)prop-2-yl]-3-oxo-androst-4-ene-17β-carboxamide;
N-[1,1,1,3,3,3-hexafluoro-2-(p-trifluoromethylphenyl)prop-2-yl]-3-oxo-androst-4-ene-17β-carboxamide;

### EXAMPLE 2

### N-(1,1,1,3,3,3-hexafluoro-2-phenyl-2-propyl)-4-methyl-3-oxo-4-aza-5α-androst-16-ene-17β-carboxamide

[compound (I), wherein the moiety AB has formula (IX), wherein R₄=Me, R₇=H, and R₁₉= Me, the C₁₆-C₁₇ bond is a double bond, R₁₈= Me, A = single bond, R₂₂=R₂₄=CF₃, R₂₃=Ph].

To a stirred mixture of 17β-cyano-4-methyl-4-aza-5α-androst-16-en-3-one (100 mg, 0.321 mmol) and 1,1,1,3,3,3-hexafluoro-2-phenyl-2-propyl trifluoromethanesulfonate (241 mg, 0.642 mmol), under nitrogen atmosphere, trifluoroacetic acid (129 mg, 1.605 mmol) was added at room temperature. The mixture was heated to 80°C for 5 hrs. After cooling in an ice bath, water (5 ml) and then a saturated aqueous solution of sodium hydrogen carbonate (5 ml) were added and the mixture was extracted with methylene chloride (2 x 5 ml). The combined organic extracts were washed with water until neutral, dried with sodium sulfate, and the solvent was evaporated under vacuum. The crude product was purified by flash chromatography (eluant: toluene/ethyl acetate/methanol 75:20:5) to yield 76 mg (42%) of the title compound.
- NMR (CDCl₃) δ:: 0.93 (s, 3H, Me (19)), 1.00 (s, 3H, Me (18)), 2.93 (s, 3H, N-Me), 3.07 (dd, 1H, H (5α)), 6.17 (s, 1H, NH ), 6.54 (m, 1H, H (16)), 7.77-7.55 (m, 5H, Ph).
- MS (FAB⁺):: 557 (M + H)⁺

Following an analogous procedure, starting from the corresponding 17β-cyano-16-unsaturated-steroids and the suitable triflate, the compounds listed below were obtained:
N-(1,1,1,3,3, 3-Hexafluoro-2-phenylprop-2-yl)-3-oxo-4-aza-5α-androst-16-ene-17β-carboxamide;
N-(1,1,1,3,3,3-Hexafluoro-2-phenylprop-2-yl)-4-methyl-3-oxo-4-aza-5α-androst-16-ene-17β-carboxamide;
N-(1,1,1-Trifluoro-2-phenylprop-2-yl)-4-methyl-3-oxo-4-aza-5α-androst-16-ene-17β-carboxamide; and
N-(1,1,1-Trifluoro-2-phenylprop-2-yl)-3-oxo-androst-4-ene-17β-carboxamide.

### EXAMPLE 3

### (a) 3-Oxo-4-aza-5α-androst-1-ene-17β-carboxamide

A solution of thionyl chloride (25 ml) in anhydrous chloroform (10 ml) was added dropwise, under nitrogen atmosphere, to a suspension of 3-oxo-4-aza-5α-androst-1-ene-17β-carboxylic acid (5.0 g) in anhydrous chloroform (250 ml), over about 30 minutes, at 0°C. After stirring at room temperature for 1 h, the volatile products were removed under reduced pressure and the white solid of 3-oxo-4-aza-5α-androst-1-ene-17β-carbonyl chloride so obtained was dissolved in anhydrous chloroform (800 ml), cooled to 0°C and treated with gaseous anhydrous ammonia for 30 minutes. After stirring the solution for 1 h at room temperature, the solvent was removed under vacuum, the residue treated with IN sodium carbonate aqueous solution (100 ml) and extracted with methylene chloride (3 x 100 ml). The combined organic extracts were dried with sodium sulfate and the solvent evaporated under reduced pressure. 5.0 g of the crude title compound were obtained.
- NMR (CDCl₃) δ:: 0.73 (s, 3H, Me (18)), 0.96 (s, 3H, Me (19)), 3.33 (dd, 1H, H (5α)), 5.25 (bs, 1H, NH (4)), 5.37 (bs, 2H, CONH₂) 5.81 (dd, 1H, H (2)), 6.80 (d, 1H, H (1)).
- NMR (DMSO) δ:: 0.59 (s, 3H, Me (18)), 0.84 (s, 3H, Me (19)), 3.18 (dd, 1H, H(5α)), 5.62 (dd, 1H, H(2)), 6.75 and 6.95 (d, 2H, CONH₂), 6.84 (d, 1H, H(1)), 7.43 (m, 1H, NH).
- IR (nujol) cm⁻¹:: 3430, 3185, 1690, 1675, 1655, 1610.

### (b) 17β-cyano-4-aza-5α-androst-1-en-3-one

[compound (II) wherein the moiety AB has formula (IX), wherein R₄=H, R₇=H, R₁₉=Me and the C₁-C₂ is a double bond, R₁₈=Me, A=single bond].

3-Oxo-4-aza-5α-androst-1-ene-17β-carboxamide (1.00 g) was added to a solution of trimethylsilylpolyphosphate (2.94 g) in chloroform (35 ml) and the mixture was refluxed for 4 hrs. After cooling, a 25% aqueous solution of sodium carbonate (100 ml) was added, the organic layer was separated and the aqueous phase was extracted with methylene chloride (3 x 100 ml). The combined organic extracts were washed with water until neutral, dried with sodium sulfate and the solvent was evaporated under vacuum. The crude product was purified by flash chromatography on silica gel (eluant: methylene chloride/acetone 70:30) to yield 580 mg of the title compound.
- NMR (CDCl₃) δ:: 0.93 (s, 3H, Me (18)), 0.98 (s, 3H, Me (19)), 3.33 (dd, 1H, H (5α)), 5.66 (bs, 1H, NH (4)), 5.81 (dd, 1H, H (2)), 6.80 (d, 1H, H (1)).
- IR (nujol) cm⁻¹:: 3400, 2240, 1670, 1597.

### (c) N-[1,1,1,3,3,3-Hexafluoro-2-phenylprop-2-yl]-3-oxo-4-aza-5α-androst-1-ene-17β-carboxamide

[compound (I) wherein the moiety AB has formula (IX), wherein R₄=H, R₇=H, R₁₉=Me and the C₁-C₂ is a double bond, R₁₈=Me, A= single bond, R₂₂=R₂₄=CF₃, R₂₃=Ph].

To a stirred mixture of 17β-cyano-4-aza-5α-androst-1-en-3-one (2.5 g) and 1,1,1,3,3,3-hexafluoro-2-phenyl-2-propyl trifluoromethanesulfonate (6.4 g), trifluoroacetic acid (3.139 ml) was added at room temperature, under nitrogen atmosphere. The reaction mixture was heated at 60°C for 3 hrs. After cooling to about 0°C, the reaction mixture is diluted with diethylether (10 ml), additioned with a saturated sodium bicarbonate aqueous solution (20 ml), and then extracted with ethyl acetate (3 x 30 ml). The combined organic extracts were washed with water until neutral, dried with sodium sulfate, and the solvent was evaporated at reduced pressure. The crude solid so obtained was purified by flash chromatography on silica gel (eluant: toluene/ethyl acetate/methanol 75:20:5) to yield 1.90 g (42%) of the title compound.
- NMR (CDCl₃) δ:: 0.76 (s, 3H, Me(18)), 0.98 (s, 3H, Me(19)), 3.33 (dd, 1H, H(5α)), 5.39 (s, 1H, NH(4)), 5.82 (dd, 2H, H(2)), 5.89 (s, 1H, NH(21)), 6.79 (d, 1H, H(1)), 7.38-7.54 (m, 5H, Ph).
- MS (FAB⁻) (m/z):: 542 [M-H]⁻, 471 [M-CHF₃-H]⁻.
- IR (nujol) cm⁻¹:: 3440, 3260, 3210, 1705, 1670, 1597.

Following an analogous procedure, starting from the corresponding 17β-cyano-4-aza-5α-androstanes and the suitable triflate, the compounds listed below were prepared:
N-(1,1,1,3,3,3-hexafluoro-2-phenylprop-2-yl)-3-oxo-4-aza-5α-androst-1-ene-17β-carboxamide;
N-(1,1,1,3,3,3-hexafluoro-2-phenylprop-2-yl)-3-oxo-4-aza-5α-androstane-17β-carboxamide;
N-(1,1,1,3,3,3-hexafluoro-2-phenylprop-2-yl)-4-methyl-3-oxo-4-aza-5α-androst-1-ene-17β-carboxamide;
N-(1,1,1,3,3,3-hexafluoro-2-phenylprop-2-yl)-4-methyl-3-oxo-4-aza-5α-androstane-17β-carboxamide;
N-[1,1,1,3,3,3-hexafluoro-2-(p-methylphenyl)prop-2-yl]-3-oxo-4-aza-5α-androst-1-ene-17β-carboxamide;
N-[1,1,1,3,3,3-hexafluoro-2-(p-fluorophenyl)prop-2-yl]-3-oxo-4-aza-5α-androst-1-ene-17β-carboxamide;
N-[1,1,1,3,3,3-hexafluoro-2-(p-chlorophenyl)prop-2-yl]-3-oxo-4-aza-5α-androst-1-ene-17β-carboxamide;
N-[1,1,1,3,3,3-hexafluoro-2-(p-trifluoromethyl)phenyl)prop-2-yl]-3-oxo-4-aza-5α-androst-1-ene-17β-carboxamide.

Analogously, starting from the corresponding 17β-cyanosteroids and triflates, the following compounds may be obtained:
17β-N-(1,1,1,3,3,3-hexafluoro-2-phenylprop-2-yl)carbamoyl-androsta-4,6-diene-3-carboxylate;
17β-N-(1,1,1,3,3,3-hexafluoro-2-phenylprop-2-yl)carbamoyl-1,3,5(10)-estratriene-3-carboxylate;
N-(1,1,1,3,3,3-hexafluoro-2-phenylprop-2-yl)-4-amino-3-oxoandrost-4-ene-17β-carboxamide;
N-(1,1,1,3,3,3-hexafluoro-2-phenylprop-2-yl)-4-amino-3-oxoandrosta-4,6-diene-17β-carboxamide;
N-(1,1,1,3,3,3-hexafluoro-2-phenylprop-2-yl)-3-oxo-6-aza-androst-4-ene-17β-carboxamide; and
N-(1,1,1,3,3,3-hexafluoro-2-phenylprop-2-yl)-3-hydroxyestra-1,3,5(10)-triene-17β-carboxamide.

### EXAMPLE 4

### N-[1,1,1,3,3,3-Hexafluoro-2-phenylprop-2-yl]-3-oxo-4-azaandrost-5-ene-17β-carboxamide

[compound (I) wherein the moiety AB has formula (IX), wherein R₄=H, R₇=H, R₁₉=Me and the C₁-C₂ is a single bond, C₅-C₆ is a double bond, H₅ is not present, R₁₈=Me, A=single bond, R₂₂=R₂₄= CF₃, R₂₃=Ph].

To a stirred mixture of 17β-cyano-4-azaandrost-5-en-3-one (2.98 g) and 1,1,1,3,3,3-hexafluoro-2-phenyl-2-propyl trifluoromethanesulfonate (7.23 g) trifluoroacetic acid (3.7 mL) is added at room temperature, under nitrogen atmosphere. The reaction mixture is heated to 60°C for 5 h. After cooling to about 0°C, the reaction mixture is diluted with methylene chloride (15 mL), a 35% NaOH solution (5mL) is added dropwise at 4°C followed by water (21 mL) and extracted with methylene chloride (2 x 15 mL). The combined organic extracts are washed with water until neutral, dried over sodium sulfate and the solvent is evaporated at reduced pressure. The crude solid so obtained is purified by flash chromatography on silica gel (eluant: ethyl acetate/n-hexane/methanol 75:20:5) to yield 912 mg of the title compound.
- NMR (CDCl₃) δ :: 0.76 (s, 3H, Me(18)), 1.13 (s, 3H, Me(19)), 2.34 (t, 1H, H(17α)), 2.46-2.52 (m, 2H, CH₂(2)), 4.82 (m, 1H, H(6)), 5.82 (s, 1H, NH(21)), 7.38 (s,1H,NH(4)), 7.35-7.55 (m, 5H, Ph).

## Claims

1. Process for preparing a compound of formula: 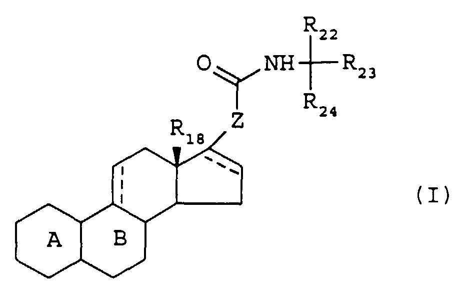 wherein the stereochemistry in position 17, if present, is α or β:
the symbols --- are, each independently, single or double bonds;
Z is a single bond, or a straight or branched C₁-C₅ alkylene;
the moiety 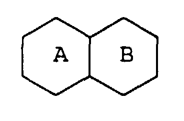 represents the A and B rings of a steroid;
R₁₈ is hydrogen or C₁-C₄ alkyl;
one of R₂₂, R₂₃ and R₂₄ is an optionally substituted C₆-C₁₀ aryl group, and the other two are C₁-C₃ perfluoroalkyl groups; said process comprising reacting a compound of formula: 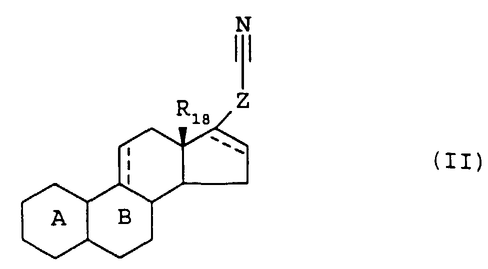 wherein the symbols --- , Z, R₁₈, and the moiety 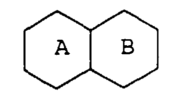 are defined as above;
with a compound of formula: 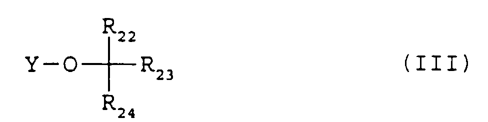 wherein R₂₂, R₂₃, and R₂₄ are defined as above, and Y is trifluoromethanesulphonyl, in the presence of a strong acid.

2. The process according to claim 1, wherein the moiety 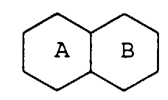 is selected from:
1) 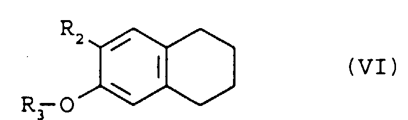 wherein: R₃ is hydrogen or C₁-C₄ alkyl; and R₂ is hydrogen or -OR₂', wherein R₂' is hydrogen or C₁-C₄ alkyl;
2) 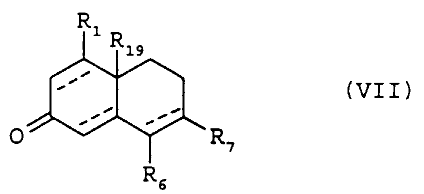 wherein: the symbols --- are, each independently, single or double bonds; R₁, R₆, R₇, and R₁₉ are, each independently, hydrogen or C₁-C₄ alkyl;
3) 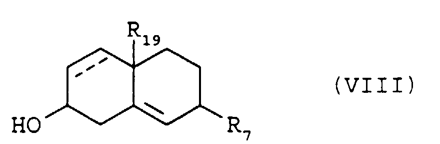 wherein: the symbol --- is a single or a double bond; R₇ is hydrogen or C₁-C₄ alkyl; and R₁₉ is hydrogen or C₁-C₄ alkyl;
4) 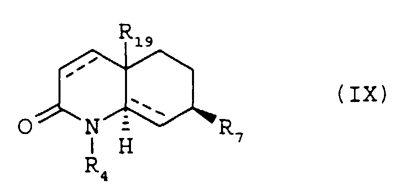 wherein: the symbols --- are, each independently, single or double bonds; R₄ is hydrogen, C₁-C₄ alkyl, C₆-C₁₀ aryl, C₇-C₁₀ arylalkyl, acetyl, benzoyl, or tosyl; R₇ is hydrogen or C₁-C₄ alkyl; R₁₉ is hydrogen or C₁-C₄ alkyl;
5) 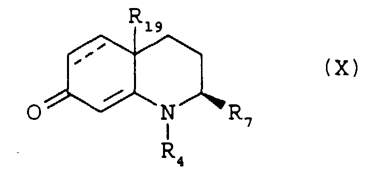 wherein: the symbol --- is a single or a double bond; R₄ is hydrogen, C₁-C₄ alkyl, C₆-C₁₀ aryl, C₇-C₁₀ arylalkyl, acetyl, benzoyl, or tosyl; R₇ is hydrogen or C₁-C₄ alkyl; R₁₉ is hydrogen or C₁-C₄ alkyl;
6) 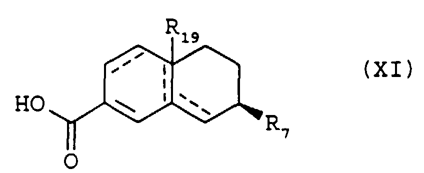 wherein: the symbols --- are, each independently, single or double bonds; R₁₉ is hydrogen, C₁-C₄ alkyl, or it is absent when linked to a double-bonded carbon atom; R₇ is hydrogen or C₁-C₄ alkyl;
7) 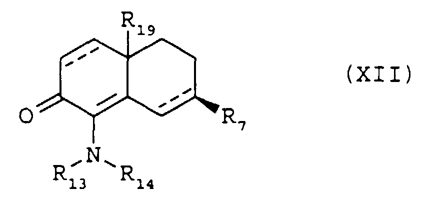 wherein: the symbols --- are, each independently, single or double bonds; R₇ is hydrogen or C₁-C₄ alkyl; R₁₉ is hydrogen or C₁-C₄ alkyl; R₁₃ and R₁₄ are, each independently, hydrogen, C₁-C₄ alkyl, C₆-C₁₀ aryl, C₇-C₁₀ arylalkyl, acetyl, benzoyl, tosyl or, taken together, phthalyl.

3. The process according to claim 1, wherein the compound of formula (I) is selected from:
N-(1,1,1,3,3,3-hexafluoro-2-phenylprop-2-yl)-3-oxo-4-aza-5α-androst-1-ene-17β-carboxamide;
N-(1,1,1,3,3,3-hexafluoro-2-phenylprop-2-yl)-3-oxo-4-aza-5α-androstane-17β-carboxamide;
N-(1,1,1,3,3,3-hexafluoro-2-phenylprop-2-yl)-4-methyl-3-oxo-4-aza-5α-androst-1-ene-17β-carboxamide;
N-(1,1,1,3,3,3-hexafluoro-2-phenylprop-2-yl)-4-methyl-3-oxo-4-aza-5α-androstane-17β-carboxamide;
N-(1,1,1,3,3,3-hexafluoro-2-phenylprop-2-yl)-3-oxo-4-aza-5α-androst-16-ene-17-carboxamide;
N-(1,1,1,3,3,3-hexafluoro-2-phenylprop-2-yl)-4-methyl-3-oxo-4-aza-5α-androst-16-ene-17-carboxamide;
N-(1,1,1,3,3,3-hexafluoro-2-phenylprop-2-yl)-3-oxo-androst-4-ene-17β-carboxamide;
N-(1,1,1,3,3,3-hexafluoro-2-phenylprop-2-yl)-3b-hydroxy-androst-5-ene-17β-carboxamide;
N-[1,1,1,3,3,3-hexafluoro-2-(p-methylphenyl)prop-2-yl]-3-oxo-4-aza-5α-androst-1-ene-17β-carboxamide;
N-[1,1,1,3,3,3-hexafluoro-2-(p-methylphenyl)prop-2-yl]-3-oxo-androst-4-ene-17β-carboxamide;
N-[1,1,1,3,3,3-hexafluoro-2-(p-fluorophenyl)prop-2-yl]-3-oxo-4-aza-5α-androst-1-ene-17β-carboxamide;
N-[1,1,1,3,3,3-hexafluoro-2-(p-fluorophenyl)prop-2-yl]-3-oxo-androst-4-ene-17β-carboxamide;
N-[1,1,1,3,3,3-hexafluoro-2-(p-chlorophenyl)prop-2-yl]-3-oxo-4-aza-5α-androst-1-ene-17β-carboxamide;
N-[1,1,1,3,3,3-hexafluoro-2-(p-chlorophenyl)prop-2-yl]-3-oxo-androst-4-ene-17β-carboxamide;
N-[1,1,1,3,3,3-hexafluoro-2-(p-trifluoromethyl)phenyl) prop-2-yl]3-oxo-4-aza-5α-androst-1-ene-17β-carboxamide;
N-[1,1,1,3,3,3-hexafluoro-2-(p-trifluoromethylphenyl) prop-2-yl] 3-oxo-androst-4-ene-17β-carboxamide;
17β-N-(1,1,1,3,3,3-hexafluoro-2-phenylprop-2-yl)carbamoyl-androsta-4,6-diene-3-carboxylate;
17β-N-(1,1,1,3,3,3-hexafluoro-2-phenylprop-2-yl)carbamoyl-1,3,5(10)-estratriene-3-carboxylate;
N-(1,1,1,3,3,3-hexafluoro-2-phenylprop-2-yl)]-3-oxo-6-aza-androst-4-ene-17β-carboxamide; and
N-(1,1,1,3,3,3-hexafluoro-2-phenylprop-2-yl)-3-hydroxyestra-1,3,5(10)-triene-17β-carboxamide.

## Patentansprüche

1. Verfahren zur Herstellung einer Verbindung der Formel: 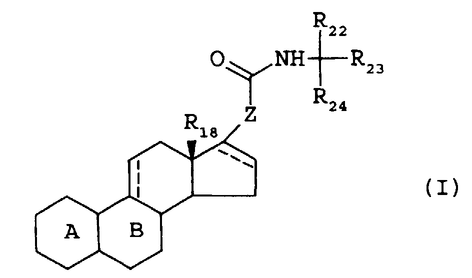 worin:
die Stereochemie an Position 17, falls vorhanden, α oder β ist;
die Zeichen --- jeweils unabhängig Einfach- oder Doppelbindungen sind;
Z eine Einfachbindung oder ein gerades oder verzweigtes C₁-C₅-Alkylen ist;
der Anteil 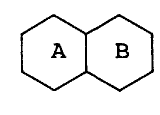 die A- und B-Ringe eines Steroids darstellen;
R₁₈ Wasserstoff oder C₁-C₄-Alkyl ist;
eine der R₂₂, R₂₃ und R₂₄ eine nach Wunsch substituierte C₆-C₁₀-Arylgruppe ist und die anderen beiden C₁-C₃-Perfluoralkylgruppen sind;
dieses Verfahren umfassend die Reaktion einer Verbindung der Formel: 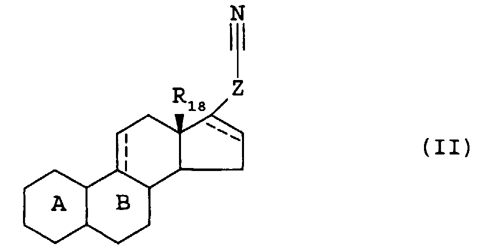 worin die Zeichen ---, Z, R₁₈ und der Anteil 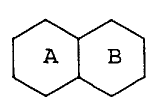 wie oben definiert sind;
mit einer Verbindung der Formel: 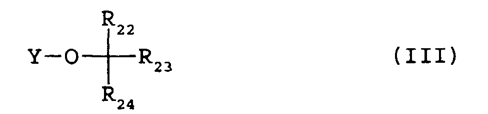 worin R₂₂, R₂₃ und R₂₄ wie oben definiert sind und Y Trifluormethansulfonyl ist, in Gegenwart einer starken Säure.

2. Verfahren nach Anspruch 1, worin der Anteil 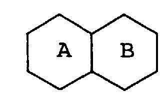 gewählt ist aus:
1) 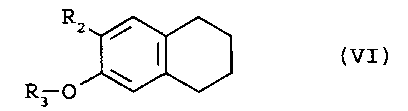 worin: R₃ Wasserstoff oder C₁-C₄-Alkyl ist; und R₂ Wasserstoff oder -OR₂' ist, worin R₂' Wasserstoff oder C₁-C₄-Alkyl ist;
2) 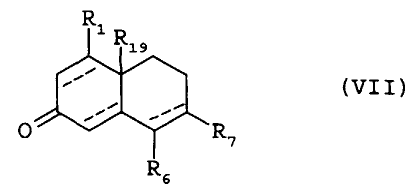 worin: die Zeichen --- jeweils unabhängig Einfach- oder Doppelbindungen sind; R₁, R₆, R₇ und R₁₉ jeweils unabhängig Wasserstoff oder C₁-C₄-Alkyl sind;
3) 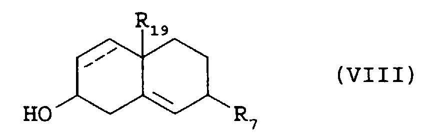 worin: das Zeichen --- eine Einfach- oder eine Doppelbindung ist; R₇ Wasserstoff oder C₁-C₄-Alkyl ist; und R₁₉ Wasserstoff oder C₁-C₄-Alkyl ist;
4) 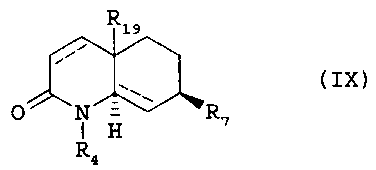 worin: die Zeichen --- jeweils unabhängig Einfach- oder Doppelbindungen sind; R₄ Wasserstoff, C₁-C₄-Alkyl, C₆-C₁₀-Aryl, C₇-C₁₀-Arylalkyl, Acetyl, Benzoyl oder Tosyl ist; R₇ Wasserstoff oder C₁-C₄-Alkyl ist; R₁₉ Wasserstoff oder C₁-C₄-Alkyl ist;
5) 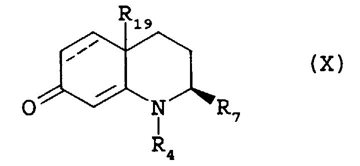 worin: das Zeichen --- eine Einfach- oder eine Doppelbindung ist; R₄ Wasserstoff, C₁-C₄-Alkyl, C₆-C₁₀-Aryl, C₇-C₁₀-Arylalkyl, Acetyl, Benzoyl oder Tosyl ist; R₇ Wasserstoff oder C₁-C₄-Alkyl ist; R₁₉ Wasserstoff oder C₁-C₄-Alkyl ist;
6) 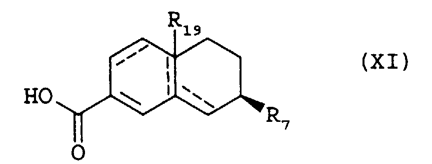 worin: die Zeichen --- jeweils unabhängig Einfach- oder Doppelbindungen sind; R₁₉ Wasserstoff, C₁-C₄-Alkyl ist oder nicht vorhanden ist bei Doppelbindung an ein Kohlenstoffatom; R₇ Wasserstoff oder C₁-C₄-Alkyl ist;
7) 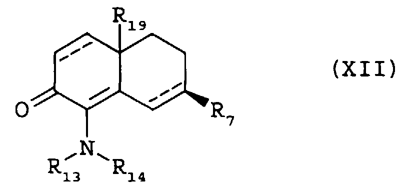 worin: die Zeichen --- jeweils unabhängig Einfach- oder Doppelbindungen sind; R₇ Wasserstoff oder C₁-C₄-Alkyl ist; R₁₉ Wasserstoff oder C₁-C₄-Alkyl ist; R₁₃ und R₁₄ jeweils unabhängig Wasserstoff, C₁-C₄-Alkyl, C₆-C₁₀-Aryl, C₇-C₁₀-Arylalkyl, Acetyl, Benzoyl, Tosyl oder zusammen genommen Phathalyl sind.

3. Verfahren nach Anspruch 1, worin die Verbindung der Formel (I) gewählt ist aus:
N-(1,1,1,3,3,3-Hexafluor-2-phenylprop-2-yl)-3-oxo-4-aza-5α-androst-1-en-17β-carboxamid;
N-(1,1,1,3,3,3-Hexafluor-2-phenylprop-2-yl)-3-oxo-4-aza-5α-androstan-17β-carboxamid;
N-(1,1,1,3,3,3-Hexafluor-2-phenylprop-2-yl)-4-methyl-3-oxo-4-aza-5α-androst-1-en-17β-carboxamid;
N-(1,1,1,3,3,3-Hexafluor-2-phenylprop-2-yl)-4-methyl-3-oxo-4-aza-5α-androstan-17β-carboxamid;
N-(1,1,1,3,3,3-Hexafluor-2-phenylprop-2-yl)-3-oxo-4-aza-5α-androst-16-en-17-carboxamid;
N-(1,1,1,3,3,3-Hexafluor-2-phenylprop-2-yl)-4-methyl-3-oxo-4-aza-5α-androst-16-en-17-carboxamid;
N-(1,1,1,3,3,3-Hexafluor-2-phenylprop-2-yl)-3-oxo-androst-4-en-17β-carboxamid;
N-(1,1,1,3,3,3-Hexafluor-2-phenylprop-2-yl)-3b-hydroxy-androst-5-en-17β-carboxamid;
N-[1,1,1,3,3,3-Hexafluor-2-(p-methylphenyl)prop-2-yl]-3-oxo-4-aza-5α-androst-1-en-17β-carboxamid;
N-[1,1,1,3,3,3-Hexafluor-2-(p-methylphenyl)prop-2-yl]-3-oxo-androst-4-en-17β-carboxamid;
N-[1,1,1,3,3,3-Hexafluor-2-(p-fluorphenyl)prop-2-yl]-3-oxo-4-aza-5α-androst-1-en-17β-carboxamid;
N-[1,1,1,3,3,3-Hexafluor-2-(p-fluorphenyl)prop-2-yl]-3-oxo-androst-4-en-17β-carboxamid;
N-[1,1,1,3,3,3-Hexafluor-2-(p-chlorphenyl)prop-2-yl]-3-oxo-4-aza-5α-androst-1-en-17β-carboxamid;
N-[1,1,1,3,3,3-Hexafluor-2-(p-chlorphenyl)prop-2-yl]-3-oxo-androst-4-en-17β-carboxamid;
N-[1,1,1,3,3,3-Hexafluor-2-(p-trifluormethylphenyl)prop-2-yl]-3-oxo-4-aza-5α-androst-1-en-17β-carboxamid;
N-[1,1,1,3,3,3-Hexafluor-2-(p-trifluormethylphenyl)prop-2-yl]-3-oxo-androst-4-en-17β-carboxamid;
17β-N-(1,1,1,3,3,3-Hexafluor-2-phenylprop-2-yl)carbamoyl-androsta-4,6-dien-3-carboxylat;
17β-N-(1,1,1,3,3,3-Hexafluor-2-phenylprop-2-yl)carbamoyl-1,3,5(19)-estratrien-3-carboxylat;
N-(1,1,1,3,3,3-Hexafluor-2-phenylprop-2-yl)-3-oxo-6-aza-androst-4-en-17β-carboxamid; und
N-(1,1,1,3,3,3-Hexafluor-2-phenylprop-2-yl)-3-hydroxyestra-1,3,5(10)-trien-17β-carboxamid.

## Revendications

1. Procédé pour la préparation d'un composé de formule: 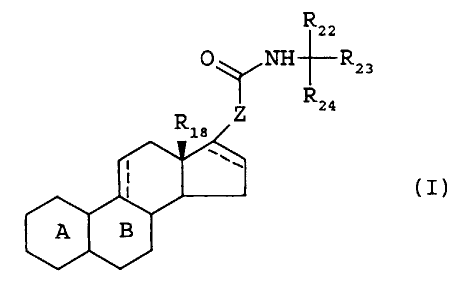 dans laquelle la stéréochimie en position 17, lorsque elle est présente, est de type α ou β:
les symboles ---- représentent, chacun indépendamment l'un de l'autre, une liaison simple ou une double liaison;
Z est une liaison simple, ou un groupe alkylène en C₁-C₅ linéaire ou ramifié;
l'entité 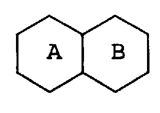 représente les cycles A et B d'un stéroide;
R₁₈ représente l'hydrogène ou un alkyle en C₁-C₄;
un des groupes R₂₂, R₂₃ et R₂₄ est un groupe aryle en C₆-C₁₀ éventuellement substitué, alors que les deux autres sont des groupes perfluoroalkyle en C₁-C₃;
le dit-procédé comprenant la mise en réaction d'un composé de formule: 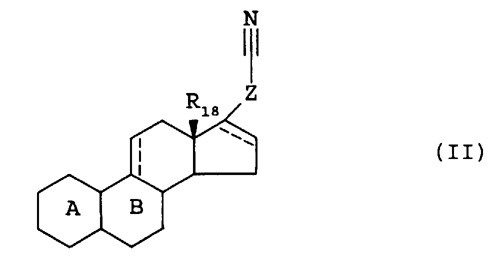 dans laquelle les symboles ---- , Z, R₁₈, et l'entité 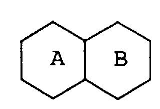 sont tels que définis ci-dessus;
avec un composé de formule: 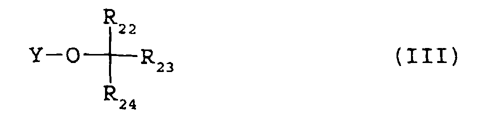 dans laquelle les groupes R₂₂, R₂₃ et R₂₄ sont tels que définis ci-dessus, et Y représente un groupe trifluorométhanesulfonyl, la réaction se faisant en présence d'un acide fort.

2. Procédé selon la revendication 1, pour lequel l'entité 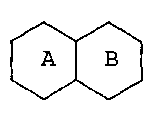 est choisie parmi:
1) 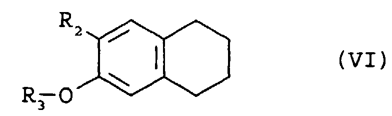 dans laquelle: R₃ est l'hydrogène ou un alkyle en C₁-C₄; et R₂ est l'hydrogène ou un groupe -OR₂', où R₂' représente l'hydrogène ou un alkyle en C₁-C₄;
2) 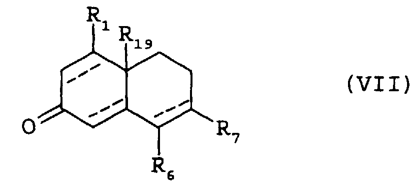 dans laquelle: les symboles ---- représentent, chacun indépendamment l'un de l'autre, une liaison simple ou double; les groupes R₁, R₆, R₇, et R₁₉ représentent, chacun indépendamment, l'hydrogène ou un alkyle en C₁-C₄;
3) 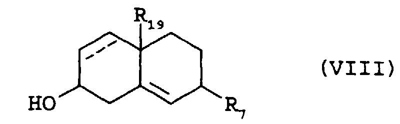 dans laquelle: le symbole ---- représente une liaison simple ou une double liaison; le groupe R₇ est l'hydrogène ou un alkyle en C₁-C₄, et R₁₉ est l'hydrogène ou un alkyle en C₁-C₄;
4) 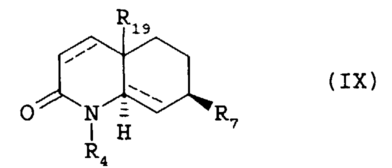 dans laquelle: les symboles ---- représentent, chacun indépendamment, des liaison simple ou double; le groupe R₄ est l'hydrogène, un alkyle en C₁-C₄, un aryle en C₆-C_{10,} un arylalkyle en C₇-C₁₀, un acétyle, un benzoyle, ou un tosyle; R₇ est l'hydrogène ou un alkyle en C₁-C₄; R₁₉ est l'hydrogène ou un alkyle en C₁-C₄;
5) 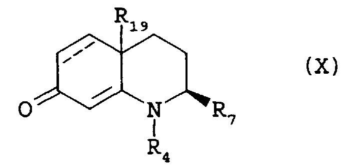 dans laquelle: le symbole ---- représente une liaison simple ou une double liaison; le groupe R₄ est l'hydrogène, un alkyle en C₁-C₄, un aryle en C₆-C₁₀, un arylalkyle en C₇-C₁₀, un acétyle, un benzoyle, ou un tosyle; R₇ est l'hydrogène ou un alkyle en C₁-C₄; R₁₉ est l'hydrogène ou un alkyle en C₁-C₄;
6) 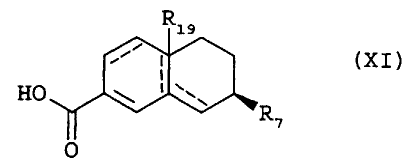 dans laquelle: les symboles ---- représentent, chacun indépendamment, des liaisons simple ou double; le groupe R₁₉ est l'hydrogène, un groupe alkyle en C₁-C₄, ou bien il est absent lorsque il est lié à un atome de carbone à double liaison; R₇ est l'hydrogène ou un groupe alkyle en C₁-C₄;
7) 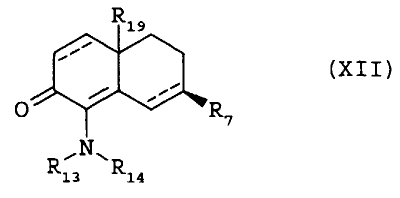 dans laquelle: les symboles ---- représentent, chacun indépendamment, des liaisons simple ou double; le groupe R₇ est l'hydrogène, un alkyle en C₁-C₄; R₁₉ est l'hydrogène ou un alkyle en C₁-C₄; R₁₃ et R₁₄ sont, chacun indépendamment, l'hydrogène, un alkyle en C₁-C₄, un aryle en C₆-C₁₀, un arylalkyle en C₇-C₁₀, un acétyle, un benzoyle, un tosyle ou bien, pris ensemble, ils constituent un groupe phthalyle.

3. Procédé selon la revendication 1, pour lequel le composé de formule (I) est choisi parmi:
N-(1,1,1,3,3,3-hexafluoro-2-phénylprop-2-yl)-3-oxo-4-aza-5α-androst-1-ène-17β-carboxamide;
N-(1,1,1,3,3,3-hexafluoro-2-phénylprop-2-yl)-3-oxo-4-aza-5α-androstane-17β-carboxamide;
N-(1,1,1,3,3,3-hexafluoro-2-phénylprop-2-yl)-4-méthyl-3-oxo-4-aza-5α-androst-1-ène-17β-carboxamide;
N-(1,1,1,3,3,3-hexafluoro-2-phénylprop-2-yl)-4-méthyl-3-oxo-4-aza-5α-androstane-17β-carboxamide;
N-(1,1,1,3,3,3-hexafluoro-2-phénylprop-2-yl)-3-oxo-4-aza-5α-androst-16-ène-17-carboxamide;
N-(1,1,1,3,3,3-hexafluoro-2-phénylprop-2-yl)-4-méthyl-3-oxo-4-aza-5α-androst-16-ène-17-carboxamide;
N-(1,1,1,3,3,3-hexafluoro-2-phénylprop-2-yl)-3-oxo-androst-4-ène-17β-carboxamide;
N-(1,1,1,3,3,3-hexafluoro-2-phénylprop-2-yl)-3b-hydroxy-androst-5-ène-17β-carboxamide;
N-[1,1,1,3,3,3-hexafluoro-2-(p-méthylphényl)prop-2-yl]-3-oxo-4-aza-5α-androst-1-ène-17β-carboxamide;
N-[1,1,1,3,3,3-hexafluoro-2-(p-méthylphényl)prop-2-yl]-3-oxo-androst-4-ène-17β-carboxamide;
N-[1,1,1,3,3,3-hexafluoro-2-(p-fluorophényl)prop-2-yl]-3-oxo-4-aza-5α-androst-1-ène-17β-carboxamide;
N-[1,1,1,3,3,3-hexafluoro-2-(p-fluorophényl)prop-2-yl]-3-oxo-androst-4-ène-17β-carboxamide;
N-[1,1,1,3,3,3-hexafluoro-2-(p-chlorophényl)prop-2-yl]-3-oxo-4-aza-5α-androst-1-ène-17β-carboxamide;
N-[1,1,1,3,3,3-hexafluoro-2-(p-chlorophényl)prop-2-yl]-3-oxo-androst-4-ène-17β-carboxamide;
N-[1,1,1,3,3,3-hexafluoro-2-(p-trifluorométhylphényl)prop-2-yl]-3-oxo-4-aza-5α-androst-1-ène-17β-carboxamide;
N-[1,1,1,3,3,3-hexafluoro-2-(p-trifluorométhylphényl)prop-2-yl]-3-oxo-androst-4-ène-17β-carboxamide;
17β-N-(1,1,1,3,3,3-hexafluoro-2-phénylprop-2-yl)-carbamoyl-androsta-4, 6-diène-3-carboxylate;
17β-N-(1,1,1,3,3,3-hexafluoro-2-phénylprop-2-yl)-carbamoyl-1,3,5(10)-estratriène-3-carboxylate;
N-(1,1,1,3,3,3-hexafluoro-2-phénylprop-2-yl)-3-oxo-6-aza-androst-4-ène-17β-carboxamide; et
N-(1,1,1,3,3,3-hexafluoro-2-phénylprop-2-yl)-3-hydroxyestra-1,3,5(10)-triène-17β-carboxamide.
